(19) Europäisches Patentamt
European Patent Office
Office européen des brevets

(11) Veröffentlichungsnummer: **0 029 618**
**B1**

# (12) EUROPÄISCHE PATENTSCHRIFT

(45) Veröffentlichungstag der Patentschrift: **14.03.84**

(51) Int. Cl.³: **C 07 D 295/02,** C 07 D 211/14, A 01 N 43/34

(21) Anmeldenummer: **80200924.1**

(22) Anmeldetag: **01.10.80**

## (54) Insektizide Mittel und Piperidinderivat.

(30) Priorität: **27.11.79 DE 2947649**

(43) Veröffentlichungstag der Anmeldung: **03.06.81 Patentblatt 81/22**

(45) Bekanntmachung des Hinweises auf die Patenterteilung: **14.03.84 Patentblatt 84/11**

(84) Benannte Vertragsstaaten: **AT BE CH DE FR GB IT LI LU NL SE**

(56) Entgegenhaltungen:
**DE - A - 2 459 129**
**DE - B - 1 032 023**
**FR - A - 1 360 329**
**FR - A - 1 604 269**
**FR - A - 2 308 364**
**US - A - 3 297 703**

**CHEMICAL ABSTRACTS, Band 69, 1968, Seite 1009, Nr. 10543t Columbus, Ohio, U.S.A. T. KURIHARA et al.: "Ulcer remedies"**
**CHEMISTRY AND INDUSTRY, Nr. 5, 5. März 1977, Seite 202 London, G.B. M. TANAKA et al.: "Base-catalysed isomerisation of N-(3,7-dimethyl-octa-2,6-dienyl)dialkylamine to N-(3,7-dimethylocta-1,3-dienyl)di-alkylamine: dihydrocitral from isoprene"**

(73) Patentinhaber: **C.F. Spiess & Sohn GmbH & Co. Chemische Fabrik, D-6719 Kleinkarlbach (DE)**

(72) Erfinder: **Himmelreich, Rolf G., Uhlandstrasse 9, D-6718 Grünstadt (DE)**
Erfinder: **Spiess, Wolfram, Dr. Dipl.-Chem., Heinrich-Becker-Strasse 16, D-6718 Grünstadt (DE)**

(74) Vertreter: **Eggert, Hans-Gunther, Dr., Räderscheidtstrasse 1, D-5000 Köln 41 (DE)**

(56) Entgegenhaltungen: (Fortsetzung)
**BULLETIN OF THE CHEMICAL SOCIETY OF JAPAN, Band 46, Nr. 1, Januar 1973, Seiten 222-225 K. TAKABE et al.: "Syntheses of isoprenoids by telomerizations. VIII. Anionic telomerizations of isoprene with secondary amines"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 618 B1

BUNDESDRUCKEREI BERLIN

Insektizide MIttel und Piperidinderivat

Gegenstand der vorliegenden Erfindung ist die Verwendung von N-1-Dodecylpyrrolidin und -piperidin sowie von N-substituierten Pyrrolidinen, Piperidinen oder Hexamethyleniminen der allgemeinen Formel

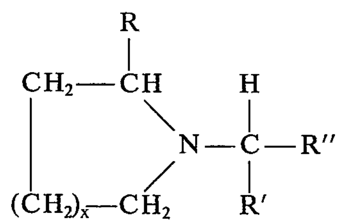

worin
x = 1, 2 oder 3;
R = H, $CH_3$ oder $C_2H_5$; sowie
a) R' = H oder $CH_3$; und
R'' ein geradkettiger aliphatischer Rest ist, wobei die Summe der Anzahl der Kohlenstoffatome in den Resten R' plus R'' 7 bis 10 beträgt, oder
b) R' = Phenyl;
R'' = $-COOR_1$, wobei $R_1$ eine $C_{1-4}$-Alkylgruppe; oder
c) R' = H;
R'' = $CH_2—NH—CH_2—CH_2—$ 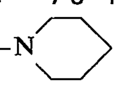 ist
als Insektizid.

Die erfindungsgemäß zu verwendenden Verbindungen sind teilweise als chemische Substanzen formelmäßig aus der DE-B-1 032 023 bekannt, wo sie als Mittel zur Bekämpfung unerwünschten Pflanzenwuchses vorgeschlagen werden.

Gegenstand der Erfindung sind ferner insektizide Mittel, enthaltend als Wirksubstanz Verbindungen der allgemeinen Formel

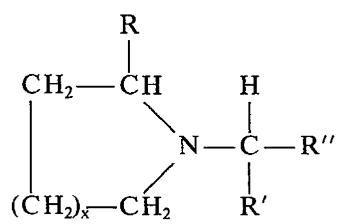

worin
x = 1, 2 oder 3;
R = H, $CH_3$ oder $C_2H_5$ und
a) R' = Phenyl und
R'' = $-COOR_1$, wobei $R_1$ eine $C_{1-4}$-Alkylgruppe oder
b) R' = H und
R'' = $CH_2—NH—CH_2—CH_2—$ 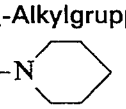 ist.

Wenn R' = Phenyl und R'' eine Carboxylgruppe ist oder wenn R' = H und

R'' = $CH_2—NH—CH_2—CH_2—$ 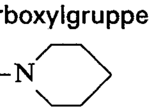

so ist x = 2 und R = H bevorzugt.

Diese heterocyclischen Amine haben nur eine geringe Toxizität auf Warmblüter. Sie sind daher sicherer zu handhaben und weniger gefährlich für Mensch und Nutztier als die meisten der bisher verwendeten Insektizide.

Wegen ihrer guten insektiziden Eigenschaften sind die Aufwandmengen den bekannten Insektiziden zumindest ebenbürtig, sie sind ihnen jedoch toxikologisch und ökonomisch gesehen überlegen.

Während die entsprechenden heterocyclischen Amine, die am Stickstoffatom nicht substituiert sind, als Insektizide unwirksam sind, erwiesen sich die entsprechenden N-substituierten Verbindungen als wirksam.

Die erfindungsgemäß zu verwendenden tertiären, heterocyclischen Amine werden hergestellt, indem man die entsprechenden heterocyclischen Amine Pyrrolidin, Piperidin oder Hexamethylenimin,

die gegebenenfalls in der 2-Stellung durch $CH_3$ oder $C_2H_5$ substituiert sind, mit entsprechenden Halogenverbindungen in bekannter Weise umsetzt.

Das Halogenid des Restes der am Stickstoffatom zu substituierenden Gruppierung, am zweckmäßigsten der Chlor- oder Bromverbindungen wird mit dem heterocyclischen Amin umgesetzt. Dabei kann man in vielen Fällen mit einem Molverhältnis von 1 : 1 arbeiten und erhält das Hydrohalogenid der gewünschten Verbindung, aus dem durch Reaktion mit Lauge das tertiäre Amin gewonnen wird. In einigen Fällen ist es ratsam, 2 Mol Amin mit 1 Mol Halogenverbindung zur Reaktion zu bringen. Man kann die dabei anfallenden heterocyclischen Amin-Hydrohalogenid-Verbindungen anschließend wieder ins Amin überführen. Anstelle eines zweiten Mols heterocyclischen Amins kann man in vielen Fällen auch ein preiswertes tertiäres Amin als Hilfsamin einsetzen, beispielsweise Triethylamin.

Die Reaktion kann mit oder ohne Lösungsmittel ausgeführt werden. In einigen Fällen muß stark gekühlt werden, häufiger ist es jedoch notwendig, die Mischung eine Zeitlang zu erwärmen, wobei in einigen Fällen bis 160°C erhitzt werden muß, um die Umsetzung zu erreichen. Man kann die erfindungsgemäß zu verwendenden tertiären, heterocyclischen Amine aber auch durch Ringbildung, z. B. aus Derivaten von Diolen und primären Aminen, erhalten.

Der Pyrrolidin-, Piperidin- oder Hexamethyleniminring kann in der 2-Position eine Methyl- oder Ethylgruppe tragen. Es kommen insbesondere in Betracht: 2-Methyl-Pyrrolidin, 2-Methyl-piperidin, 2-Ethyl-piperidin.

Der am Stickstoffatom des heterocyclischen Amins gebundene Rest CHR'R'' ist vorzugsweise ein Alkylrest mit 8 bis 11 Kohlenstoffatomen, z. B. ein Octyl-, Nonyl-, Decyl oder Undecylrest, ein Phenylessigsäurerest, der mit einer Alkylgruppe mit 1 bis 4 Kohlenstoffatomen verestert ist, vorzugsweise mit einem Methyl- oder Ethylrest, oder der Rest

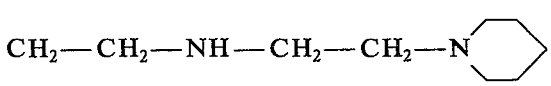

Einige der erfindungsgemäßen Insektizide wirken in Kombination mit bekannten Insektiziden wie Pyrethroiden, Carbamaten und Phosphorsäurederivaten synergistisch. Dies ermöglicht in der Praxis das Arbeiten mit besonders niedrigen Aufwandmengen.

Der Synergismus der Amine kann dadurch nachgewiesen werden, daß sie mit bekannten Insektiziden in Konzentrationen angewandt werden, bei denen sich gerade erste insektizide Wirkungen zeigen. Durch den Zusatz der Amine wird die Wirkung der bekannten Insektizide beim Auftreten von Synergismus gesteigert. Auch Verbindungen mit starker insektizider Wirkung, wie die Decyl- und Undecyl-Verbindungen des Piperidins und des Pyrrolidins oder die Phenylessigsäure-ethylester-derivate dieser beiden Amine zeigen im Gemisch mit bekannten Insektiziden eine synergistische Wirkung, die allerdings erst bei entsprechend starker Verdünnung nachweisbar ist.

Die Wirksamkeit der tertiären, heterocyclischen Amine wird durch eine Vielzahl von Beispielen deutlich gemacht. Als Versuchstiere wurden dabei Kornkäfer (sitophilus granarius L) und Taufliegen (drosophila melanogaster) eingesetzt.

Für die Versuche zur Überprüfung der Potenzierungswirkung wurden Cypermethrin und Permethrin als Vertreter der Pyrethroide, Dimethoat als Vertreter der Phosphorester und Dimetilan als Vertreter der Carbamate eingesetzt.

Cypermethrin:

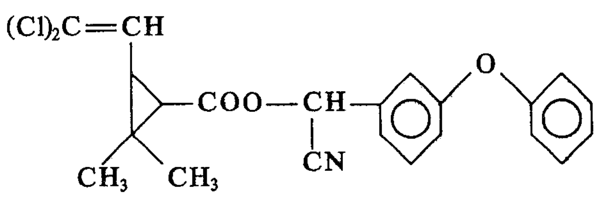

Permethrin:

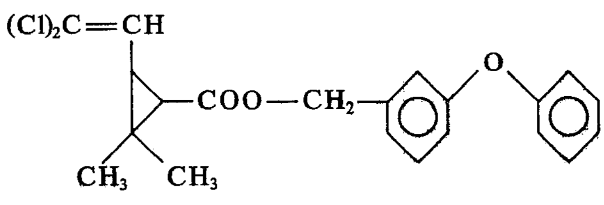

Dimethoat:

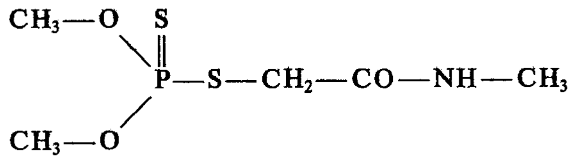

Dimetilan:

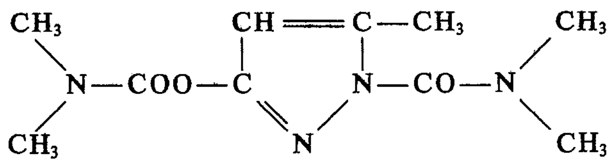

Das Mengenverhältnis von eingesetztem Insektizid zu tertiärem, heterocyclischem Amin kann zwischen 1 : 0,2 und 1 : 100 liegen; wobei sich ein Verhältnis zwischen 1 : 5 und 1 : 10 als besonders günstig erwies. Bei gleicher Wirksamkeit kann das Insektizid in vielen Fällen mit 1/10 bis 1/25 der in der Praxis üblichen Aufwandmenge eingesetzt werden.

Wie aus den Beispielen und Tabellen ersichtlich ist, gibt es in der Reihe der tertiären, heterocyclischen Amine solche mit insektizider Wirkung sowie solche mit insektizider und synergistischer Wirkung.

Die Erfindung wird im folgenden durch Beispiele näher erläutert.

Beispiel 1

Herstellung von N-1-Nonyl-2-ethyl-piperidin

20,7 g 1-Bromnonan wurden mit 22,6 g 2-Ethyl-piperidin versetzt. Die Mischung wurde eine Stunde lang am Rückflußkühler mit Natronkalkrohr im siedenden Wasserbad erhitzt. Nach dem Abkühlen wurde im Scheidetrichter 2mal mit je 50 ml Wasser ausgeschüttelt. Die organische Phase wurde im Vakuum fraktioniert. Es wurden 8,8 g einer schwach gelblich gefärbten Flüssigkeit erhalten, die bei 121°C bei $6{,}65 \cdot 10^{-4}$ bar destillierte. Bei einer acidimetrischen Titration wurde ein Tritrationswert gefunden, der einem Äquivalentgewicht von 232,8 entspricht (berechnet 239,4).

Beispiel 2

Insektizide Eigenschaften von tertiären, heterocyclischen Aminen, getestet an Drosophila melanogaster

Die Versuche wurden in Petrischalen von 10 cm Durchmesser ausgeführt. Die Aminkomponenten wurden mit 1 ml 0,05%iger Lösung + 1 ml Aceton pro Petrischale getestet. In den Petrischalen wurden die Lösungen am Boden gut verteilt und 30 min lang antrocknen lassen. In jede Petrischale wurden 20 mit Kohlendioxid betäubte Drosophila eingezählt. Alle 5 min, insgesamt 12mal, wurde die Anzahl der toten Drosophila ausgezählt und notiert. Die Zahlen wurden aufsummiert und im Verhältnis zur möglichen Zahl an toten Drosophila in Prozent angegeben, wobei der Tod aller Drosophila in den ersten 5 min 100% Wirkung entspricht. Jeder Versuch wurde mit 3 Wiederholungen durchgeführt. Die Versuche wurden bei sonst stets gleichbleibenden Bedinungen bei Temperaturen von 20 – 22°C durchgeführt. Die Kontrollversuche zeigten bei Versuchsabschluß stets 0% Mortalität. In Tabelle 1 sind die Mittelwerte der Ergebnisse zusammengefaßt.

Tabelle 1

Insektizide Wirkungen tertiärer, heterocyclischer Amine an Drosophila melanogaster (Taufliegen)

| Substanz | Wirkung in % |
|---|---|
| Kontrolle | 0 |
| N-1-Undecyl-pyrrolidin | 76,7 |
| 2-N-Pyrrolidino-undecan | 76,7 |
| N-1-Decyl-piperidin | 87,9 |
| N-1-Undecyl-piperidin | 57,9 |
| N-1-Decyl-2-methyl-piperidin | 61,3 |
| N-1-Decyl-hexamethylenimin | 49,2 |
| N-1-Octyl-2-ethyl-piperidin | 97,5 |
| N-1-Nonyl-2-ethyl-piperidin | 86,7 |

## Beispiel 3

### Insektizide Eigenschaften von tertiären, heterocyclischen Aminen, getestet an Kornkäfern (sitophilus granarius L.)

In Petrischalen von 15 cm Durchmesser mit talcumbepuderten Seitenwänden, um ein Entfliehen der Kornkäfer zu verhindern, werden jeweils 2 ml der Acetonlösungen der tertiären, heterocyclischen Amine einpipettiert und durch vorsichtiges Schwenken der Schalen gleichmäßig auf dem Boden verteilt. Nach dem Abdampfen des Acetons und dem Antrocknen der Beläge wurden in jede Schale 25 Kornkäfer eingezählt. Nach einer Stunde wurde jeweils ein 12,5 cm-Rundfilter in die Schalen eingelegt, um den Käfern das Befreien aus der Rückenlage zu ermöglichen, was auf der glatten Glasfläche nicht möglich ist. Die lichtscheuen Käfer verkrochen sich mit Vorliebe unter die Filter und blieben so ständig mit den Wirkstoffbelägen in Kontakt. Alle Versuche liefen mit 3 Wiederholungen. Nach 3 Stunden wurden die toten Käfer ausgezählt. In der Tabelle 2 ist jeweils der Durchschnitt der Summe der 4 Einzelwerte in Prozent angegeben. 25 tote Kornkäfer entsprechen dabei einer Wirkung von 100%. Die Temperaturen betrugen während der Tests 19–21° C. Die zu prüfenden Amine wurden zunächst mit 0,5 mg Wirkstoff pro ml als Acetonlösungen eingesetzt. Die Konzentrationen betrugen also 0,05%, später wurden auch niedrigere Konzentrationen angewendet. Auch bei diesen Tests zeigten die Kontrollversuche stets bei Versuchsende eine Mortalität der Kornkäfer von 0%. Die Ergebnisse sind in der Tabelle 2 zusammengestellt.

Tabelle 2

Insektizide Wirkungen tertiärer, heterocyclischer Amine an Kornkäfern (sitophilus granarius L.)

| Substanz | Konzentration in % | Wirkung in % |
|---|---|---|
| Kontrolle | 0 | 0 |
| N-1-Decyl-pyrrolidin | 0,05 | 81,6 |
| N-1-Undecyl-pyrrolidin | 0,05 | 100 |
| N-1-Undecyl-pyrrolidin | 0,025 | 24,0 |
| N-1-Dodecyl-pyrrolidin | 0,05 | 89,3 |
| 2-N-Pyrrolidino-undecan | 0,05 | 56,0 |
| N-1-Nonyl-peperidin | 0,05 | 90,8 |
| N-1-Decyl-piperidin | 0,05 | 100 |
| N-1-Decyl-piperidin | 0,025 | 100 |
| N-1-Decyl-piperidin | 0,0125 | 96,0 |
| N-1-Decyl-piperidin | 0,00625 | 13,2 |
| N-1-Undecyl-piperidin | 0,05 | 100 |
| N-1-Undecyl-piperidin | 0,025 | 86,8 |
| N-1-Undecyl-piperidin | 0,0125 | 10,8 |
| 2-N-Piperidino-undecan | 0,05 | 98,8 |
| 2-N-Piperidino-undecan | 0,025 | 89,2 |
| 2-N-Piperidino-undecan | 0,0125 | 13,2 |
| N-1-Decyl-hexamethylenimin | 0,05 | 69,2 |
| N-1-Decyl-2-methyl-piperidin | 0,05 | 90,8 |
| N-1-Nonyl-2-ethyl-piperidin | 0,05 | 90,8 |

## Beispiel 4

### Die insektiziden Wirkungen von tertiären, heterocyclischen Aminen und die synergistischen Eigenschaften mit dem Phosphorsäureester Dimethoat und dem Carbamat Dimetilan, getestet an Drosophila melanogaster

Die Versuche wurden in Petrischalen von 10 cm Durchmesser ausgeführt. Es wurden zunächst die gerade noch wirksamen Konzentrationen von Dimethoat und Dimetilan bestimmt. Für Dimethoat wurde 0,005% und für Dimetilan 0,0001% als günstige Versuchskonzentration ermittelt. Die Aminkomponente wurde mit 1 ml 0,05%iger Lösung + 1 ml Aceton auf insektizide Wirkung getestet. Zur Bestimmung des Synergismus wurde 1 ml 0,05%ige Aminlösung + 1 ml 0,005%ige Dimethoatlösung bzw. + 1 ml 0,001%ige Dimetilanlösung pro Petrischale eingesetzt. Alle Verdünnungen wurden mit Aceton als Lösungsmittel hergestellt.

In den Petrischalen wurden die Lösungen am Boden gut verteilt und 30 min lang antrocknen lassen. In jede Petrischale wurden 20 mit Kohlendioxid betäubte Drosophila eingezählt. Alle 5 min, insgesamt 12mal, wurde die Anzahl der toten Drosophila ausgezählt und notiert. Die Zahlen wurden aufsummiert

und im Verhältnis zur möglichen Zahl an toten Drosophila in Prozent angegeben, wobei der Tod aller Drosophila innerhalb der ersten 5 min 100% Wirkung entspricht. Jeder Versuch wurde mit 3 Wiederholungen durchgeführt. In Tabelle 3 sind die Mittelwerte der Ergebnisse zusammengestellt.

Zur Ermittlung des synergistischen Faktors wurden die Einzelwerte pro Zeiteinheit durch die für Dimethoat bzw. Dimetilan für die jeweilige Zeiteinheit gefundenen Werte dividiert.

Die Werte für jede Zeiteinheit wurden summiert und durch die Anzahl der Zeiteinheiten dividiert. Die Versuche wurden bei sonst stets gleichbleibenden Bedingungen bei Temperaturen von 20–22°C ausgeführt. Die Kontrollversuche zeigten bei Versuchsabschluß stets 0% Mortalität. Hat die Substanz selbst eine insektizide Wirkung, so wurden die Wirkungsfaktoren für Dimethoat und Dimetilan nach Abzug des Substanzwertes berechnet und in der Tabelle aufgeführt.

Tabelle 3

Tests an Drosophila melanogaster

Wirksamkeit von Dimethoat: 8,6%

Wirksamkeit von Dimetilan: 2,5%

| Name der Substanz | Wirksamkeit in % | | | Faktor der Wirkungssteigerung | |
|---|---|---|---|---|---|
| | Substanz allein | Substanz +Dimethoat | Substanz +Dimetilan | bei Dimethoat | bei Dimetilan |
| N-1-Undecyl-pyrrolidin | 76,7 | 75,0 | 71,7 | – | – |
| N-2-Undecyl-pyrrolidin | 76,7 | 84,2 | 83,3 | – | – |
| N-1-Decyl-piperidin | 87,9 | 87,1 | 87,9 | – | – |
| N-1-Undecyl-piperidin | 57,9 | 62,1 | 63,8 | – | 2,36 |
| N-Decyl-2-methyl-piperidin | 61,3 | 66,7 | 77,9 | – | 2,87 |
| N-Decyl-hexamethylenimin | 49,2 | 65,0 | 68,8 | 1,84 | 7,84 |
| N-1-Octyl-2-ethyl-piperidin | 97,5 | 98,3 | 100,0 | – | – |
| N-1-Nonyl-2-ethyl-piperidin | 86,7 | 92,5 | 88,8 | – | – |
| 6-N-Pyrrolidino-undecan | 39,6 | 54,2 | 75,8 | 1,70 | 14,48 |
| N-1-Decyl-2-ethyl-piperidin | 35,0 | 55,8 | 56,3 | 2,42 | 8,52 |

## Beispiel 5

### Test von tertiären, heterocyclischen Aminen allein und im Gemisch mit Pyrethroiden an Kornkäfern (sitophilus granarius L)

In Petrischalen mit 15 cm Durchmesser mit talcumbepuderten Seitenwänden, um das Entfliehen der Kornkäfer zu verhindern, wurden jeweils 2 ml der Acetonlösungen einpipettiert und durch vorsichtiges Schwenken der Schalen gleichmäßig auf dem Boden verteilt. Nach dem Abdampfen des Acetons und dem Antrocknen der Beläge wurden in jede Schale 25 Kornkäfer eingezählt. Nach einer Stunde wurde jeweils ein 12,5 cm-Rundfilter in die Schalen eingelegt, um den Käfern das Befreien aus der Rückenlage zu ermöglichen, was auf der glatten Glasplatte nicht möglich ist. Die lichtscheuen Käfer verkrochen sich mit Vorliebe unter die Filter und blieben so ständig mit den Wirkstoffbelägen in Kontakt. Alle Versuche liefen mit 3 Wiederholungen. Nach 3 h wurden die toten Käfer ausgezählt.

In der Tabelle 4 ist jeweils der Durchschnitt der Summe der 4 Einzelwerte angegeben. 25 tote Kornkäfer entsprechen dabei einer Wirkung von 100%. Die Temperaturen betrugen während der Tests im Durchschnitt 19 bis 21°C. Der synergistische Faktor berechnet sich analog wie in Beispiel 4 angegeben.

Die zu prüfenden tertiären, heterocyclischen Amine wurden mit 1 mg Wirkstoff pro ml als

Acetonlösungen angesetzt. Sie wurden im Verhältnis 1 : 1 mit Cypermethrin bzw. Permethrin, die mit 0,01 mg Wirkstoff pro ml als Acetonlösung vorlagen, gemischt. Die Einzelkomponenten wurden im Verhältnis 1 : 1 mit Aceton gemischt.

In der Tabelle 4 sind die Wirkstoffe nach der Art des heterocyclischen Amins geordnet.

Auch bei diesen Versuchen zeigten die Kontrollversuche stets bei Versuchsende eine Mortalität der Kornkäfer von 0%.

Tabelle 4

Tests an sitophilus granarius L

Wirksamkeit von Ripcord: 31,5%

Wirksamkeit von Talcord: 9,7%

| Name der Substanz | Wirksamkeit in % | | | Synergistischer Faktor | |
|---|---|---|---|---|---|
| | Substanz allein | Substanz +Ripcord | Substanz +Talcord | bei Ripcord | bei Talcord |
| N-1-Decyl-pyrrolidin | 81,6 | 100,0 | 100,0 | – | 1,90 |
| N-1-Undecyl-pyrrolidin | 100,0 | 100,0 | 98,8 | – | – |
| N-2-Undecyl-pyrrolidin | 56,0 | 98,8 | 64,0 | 1,36 | – |
| N-1-Dodecyl-pyrrolidin | 89,3 | 92,0 | 96,0 | – | – |
| N-1-Nonyl-piperidin | 90,8 | 93,2 | 100,0 | – | – |
| N-1-Decyl-piperidin | 100,0 | 100,0 | 100,0 | – | – |
| N-1-Undecyl-piperidin | 100,0 | 100,0 | 100,0 | – | – |
| N-2-Undecyl-piperidin | 98,8 | 100,0 | 100,0 | – | – |
| N-1-Dodecyl-piperidin | 24,0 | 94,6 | – | 2,24 | – |
| Phenyl-N-piperidin-essigsäure-ethylester | 100,0 | 98,8 | 100,0 | – | – |
| N-1-Nonyl-hexamethylenimin | 40,0 | 97,2 | 84,0 | 1,82 | 4,54 |
| N-1-Decyl-hexamethylenimin | 69,2 | 93,2 | 62,8 | 1,35 | – |
| N-1-Decyl-2-methyl-piperidin | 90,8 | 100,0 | 100,0 | – | – |
| N-1-Octyl-2-ethyl-piperidin | 26,8 | 74,8 | 52,0 | 1,52 | 2,60 |
| N-1-Nonyl-2-ethyl-piperidin | 90,8 | 98,8 | 65,2 | – | – |

**Patentansprüche**

1. Verwendung von N-substituierten Pyrrolidinen, Piperidinen oder Hexamethyleniminen der allgemeinen Formel

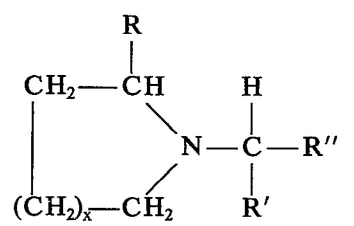

worin
$x = 1$, 2 oder 3;
$R = H$, $CH_3$ oder $C_2H_5$; sowie
a) $R' = H$ oder $CH_3$; und
$R''$ ein geradkettiger aliphatischer Rest ist, wobei die Summe der Anzahl der Kohlenstoffatome in den Resten $R'$ plus $R''$ 7 bis 10 beträgt, oder
b) $R'$ = Phenyl;
$R'' = -COOR_1$, wobei $R_1$ eine $C_{1-4}$-Alkylgruppe; oder
c) $R' = H$;
$R''$ = 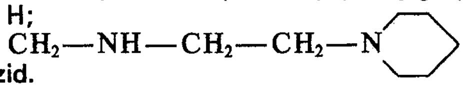 ist
als Insektizid.

2. Verwendung von N-1-Dodecylpyrrolidin oder -piperidin als Insektizid.

3. Insektizides Mittel, enthaltend als Wirksubstanz Verbindungen der allgemeinen Formel

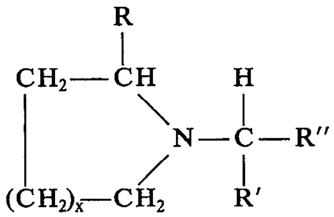

in der
$x = 1$, 2 oder 3;
$R = H$, $CH_3$ oder $C_2H_5$; sowie
a) $R'$ = Phenyl;
$R'' = -COOR_1$, wobei $R_1$ eine $C_{1-4}$-Alkylgruppe, oder
b) $R' = H$;
$R''$ = 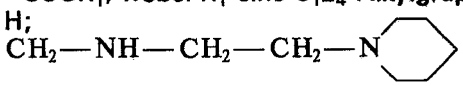 ist.

4. Insektizide Mittel nach Anspruch 3, dadurch gekennzeichnet, daß $R'$ = Phenyl und $R'' = COOR_1$ ist, wobei $R_1$ eine C-1- bis -4-Alkylgruppe ist und $x = 2$ und $R = H$ ist.

5. Insektizide Mittel nach Anspruch 3 und 4, dadurch gekennzeichnet, daß $R_1$ eine Methyl- oder Äthylgruppe ist.

6. Insektizide Mittel nach Anspruch 3, dadurch gekennzeichnet, daß $R' = H$ und

$R''$ = 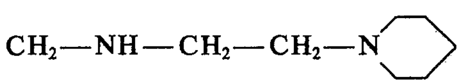

und $x = 2$ und $R = H$ ist.

7. Insektizide Mittel nach Anspruch 3 bis 6, enthaltend ein insektizides Pyrethroid.

8. Insektizide Mittel nach Anspruch 3 bis 6, enthaltend ein insektizides Carbamat.

9. Insektizide Mittel nach Anspruch 3 bis 6, enthaltend ein insektizides Phosphorsäurederivat.

10. N-1-Nonyl-2-ethyl-piperidin.

**Claims**

1. The use as an insecticide of N-substituted pyrrolidines, piperidines or hexamethyleneimines of the general formula

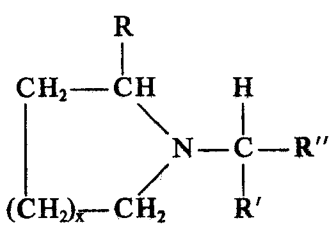

where
$x = 1$, 2 or 3;
$R = H$, $CH_3$ or $C_2H_5$;
as well as

a) R' = H or $CH_3$; and
R'' is a straight chain aliphatic radical, where the sum of the number of carbon atoms in the radicals R' plus R'' is 7 to 10, or
b) R' = phenyl;
R'' = $-COOR_1$, where $R_1$ is a $C_{1-4}$ alkyl radical; or
c) R' = H;
R'' = 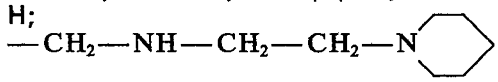 .

2. The use of N-1-dodecylpyrrolidine and -piperidine as insecticide.

3. Insecticidal agent containing as effective substance compounds of the general formula

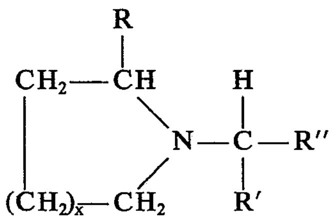

in which
x = 1, 2 or 3;
R = H, $CH_3$ or $C_2H_5$;
as well as
a) R' = phenyl;
R'' = $-COOR_1$, where $R_1$ is a $C_{1-4}$ alkyl radical; or
b) R' = H;
R'' = 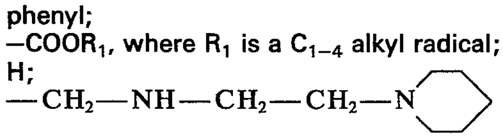 .

4. Insecticidal agent according to Claim 3, characterised in that R' = phenyl and R'' = $COOR_1$ where is a $C_{1-4}$ alkyl radical and x = 2 and R = H.

5. Insectidical agent according to Claim 3 or 4, characterised in that $R_1$ is a methyl or ethyl radical.

6. Insecticidal agent according to Claim 3, characterised in that R' = H and

R'' = 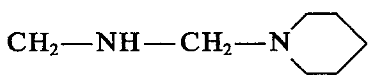

and x = 2 and R = H.

7. Insecticidal agent according to Claims 3 to 6, containing an insecticidal pyrethroid.

8. Insecticidal agent according to Claims 3 to 6, containing an insecticidal carbamate.

9. Insecticidal agent according to Claims 3 to 6, containing an insecticidal phosphoric acid derivate.

10. N-1-Nonyl-2-ethyl-piperidine.

**Revendications**

1. Utilisation de pyrrolidines, pipéridines ou hexaméthylèneimines N-substituées de formule générale

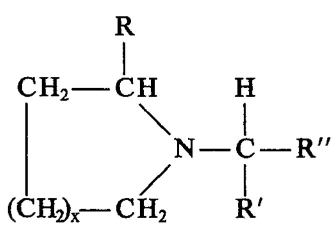

dans laquelle
x = 1, 2 ou 3;
R = H, $CH_3$ ou $C_2H_5$; et
a) R' = H ou $CH_3$; et
R'' est un reste aliphatique à chaîne droite, la somme des nombres d'atomes de carbone dans les restes R' et R'' étant de 7 à 10, ou

b) R′ = phényle;
R″ = $COOR_1$, où $R_1$ este un groupe alkyle en $C_{1-4}$; ou
c) R′ = H;
R″ = $CH_2$—NH—$CH_2$—$CH_2$—N 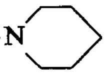 .
comme insecticides.

2. Utilisation de N-1-dodécylpyrrolidine et -pipéridine comme insecticides.

3. Agent insecticide contenant comme substance active des composés de formule générale

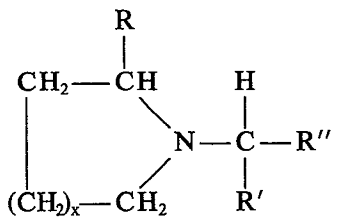

dans laquelle
x = 1, 2 ou 3;
R = H, $CH_3$ ou $C_2H_5$; et
a) R′ = phényle;
R″ = $COOR_1$, où $R_1$ est un groupe alkyle en $C_{1-4}$; ou
b) R′ = H; et
R″ = $CH_2$—NH—$CH_2$—$CH_2$—N 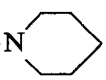 .

4. Agents insecticides selon la revendication 3, caractérisés en ce que R′ = phényle et R″ = $COOR_1$, où $R_1$ est un groupe alkyle en $C_1 - C_4$ et x = 2 et R = H.

5. Agents insecticides selon les revendications 3 et 4, caractérisés en ce que $R_1$ est un groupe méthyle ou éthyle.

6. Agents insecticides selon la revendication 3, caractérisés en ce que R′ = H et

R″ = $CH_2$—NH—$CH_2$—$CH_2$—N 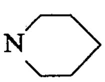

et x = 2 et R = H.

7. Agents insecticides selon les revendications 3 à 6, contenant un pyréthroïde insecticide.

8. Agents insecticides selon les revendications 3 à 6, contenant un carbamate insecticide.

9. Agents insecticides selon les revendications 3 à 6, contenant un dérivé d'acide phosphorique insecticide.

10. La N-1-nonyl-2-éthyl-piperidine.